# EUROPÄISCHE PATENTSCHRIFT

(11) **EP 1 589 918 B1**
(45) Veröffentlichungstag und Bekanntmachung des Hinweises auf die Patenterteilung: **17.10.2007**
(21) Anmeldenummer: 04708356.3
(22) Anmeldetag: 05.02.2004
(51) Int. Cl.: A61F 13/00, A61N 1/18, A61N 1/36, A61N 1/04

(54) **MEHRSCHICHTIGE KOMBINATION AUS ELEKTROSTIMULATIONSELEKTRODE UND WUNDVERBAND**
MULTI-LAYER COMBINATION OF AN ELECTRIC STIMULATION ELECTRODE AND A WOUND DRESSING
COMBINAISON MULTICOUCHE D'UNE ELECTRODE DE STIMULATION ELECTRIQUE ET D'UN PANSEMENT

(30) Priorität: 07.02.2003 DE 20301973 U
(43) Veröffentlichungstag der Anmeldung: 02.11.2005
(73) Patentinhaber: Gerromed Pflege- und Medizintechnik GmbH & Co. KG, 22547 Hamburg (DE)
(72) Erfinder: GARBET, Luca, 22605 Hamburg (DE)
(74) Vertreter: Glaeser, Joachim
(86) Internationale Anmeldenummer: PCT/EP2004/001059
(87) Internationale Veröffentlichungsnummer: WO 2004/069088

(56) Entgegenhaltungen:
- WO-A-02/068045
- US-A1- 2002 151 951
- US-B1- 6 263 226

## Beschreibung

Die Erfindung geht aus von einem Wundverband mit mindestens einem Energieübertragungsmedium (11,12), einer elektrischen Wundauflage (13) und einer Schutz/Fixierungsschicht (10).

Ein derartiger Wundverband ist bekannt (US2002/0151951 A1), er hat jedoch keine homogene Stromverteilung, so dass der Wundheilungsvorgang nicht optimal ist.

Eine Wundauflage ist bekannt (WO 02/068045 A), die aus einer Kombination eines Energieübertragungsmediums und einem elektrisch und thermisch leitendem Wundauflagemittel gebildet ist, dennoch ist die Stromverteilung nicht optimal, da in der Nähe der Kontaktstellen, an denen der Strom der jeweiligen Elektrode zugeführt wird, Strommaxima auftreten können, wodurch eine gleichmäßige Elektrostimulation der Wunde nicht sichergestellt werden kann. Daher können insbesondere großflächige Wunden nicht gleichmäßig behandelt werden.

Aus der EP 0 504 715 A1 ist eine Wundbehandlungsvorrichtung beschrieben, welche aus einer eine Öffnung aufweisenden elektrisch nicht leitfähigen Schicht und einem elektrisch leitfähigen nicht metallischen Mittel besteht. Zudem weist die Wundbehandlungsvorrichtung eine im Wesentlichen nicht haftende Wundkontaktschicht auf.

Es ist eine Elektrode zur Herstellung eines elektrischen Kontakts mit der Haut eines Patienten bekannt (US - B1- 6 263 226), die elektrisch leitende Gel-Schichten und einen Schwamm in Form eines Schichtaufbaus aufweist.

Es ist Aufgabe der vorliegenden Erfindung, einen Wundverband der eingangsgenannten Art bereitzustellen, mit dem eine gleichmäßige Elektrostimulation der Wunde erreicht und der Wundheilungsprozess erheblich verbessert werden kann.

Zur Lösung dieser Aufgabe wird ein Wundverband mit den folgenden Merkmalen vorgeschlagen:

Wundverband mit mindestens einem Energieübertragungsmedium, einer elektrisch leitenden Wundauflage und einer Schutz/Fixierungsschicht, dadurch gekennzeichnet, dass das Energieübertragungsmedium eine Folie ist, deren erste Schicht die Energiezuführung mit einer homogenen Stromverteilung über die ganze Fläche des Energieübertragungsmediums gewährleistet und deren zweite Schicht zur Energieverteilung vorgesehen ist,
Alle Merkmale des Oberbegriffs von Anspruch 1 sind von der Offenlegung US2002/0151951 A1 bekannt
die erste Schicht einen geringeren und die zweite Schicht einen höheren elektrischen Widerstand aufweist, die erste Schicht an der der zweiten Schicht abgewandten Seite die Schutz/Fixierungssicht aufweist,
die zweite Schicht an der der ersten Schicht abgewandten Seite die Wundauflage aufweist, und dass die Wundauflage wärmeleitend und/oder bei Energiebeaufschlagung wärmeerzeugend ist.

Bei dem Wundverband gemäß der Erfindung ist das Energieübertragungsmedium als Folie ausgestaltet. Diese hat eine Energiezuführungs- und eine Energieverteilungsschicht, wobei durch Aufteilung dieser Funktionen eine homogene Stromverteilung in der Wunde erreicht wird. Diese Wirkung wird noch dadurch gesteigert, dass die erste Schicht einen geringeren und die zweite Schicht einen höheren elektrischen Widerstand hat. Durch eine derartige Ausgestaltung wird über die gesamte Fläche des Energieübertragungsmediums eine homogene Stromverteilung erreicht, wobei die erste, einen geringeren elektrischen Widerstand aufweisende Schicht für eine hohe Homogenität sorgt, was weiterhin noch dadurch verstärkt wird, dass die Wundauflage wärmeleitende Eigenschaften hat und darüber hinaus bei Energiebeaufschlagung wärmeerzeugend sein kann.

Im Gegensatz zu den bekannten Wundverbänden ist es mit dem Wundverband gemäß der Erfindung möglich, auf der gesamten Fläche des Wundverbandes eine nahezu gleichmäßige energiebeaufschlagte Stimulationsbehandlung der Wunde zu erzielen, wodurch der Wundheilungsprozess erheblich verbessert wird.

Bei dem Wundverband gemäß der Erfindung weist die erste Schicht an der der zweiten Schicht abgewandten Seite eine Schutz/Fixierschicht und die zweite Schicht an der der ersten Schicht abgewandten Seite die Wundauflage auf, welche also direkt an der zu behandelten Wunde anliegt. Ein Vorteil hiervon ist, dass die Wundauflage, welche zum Schutz und der Versorgung der Wundfläche dient, im Gegensatz zu den bekannten Wundverbänden nicht extra für eine Stimulationsbehandlung entfernt werden muss, was für den Heilungsprozess der Wunde nicht förderlich wäre. Denn jeder Wechsel des Wundverbandes ist stets - trotz sorgsamer Reinigung der Wunde - mit einem Infektionsrisiko verbunden. Der Wundverband gemäß der Erfindung verbleibt hingegen auf der zu therapierenden Wunde, auch wenn keine Stimulationsbehandlung durchgeführt wird. Neben der Einsparung von Verbandsmaterial wird zusätzlich eine erhebliche Reduzierung der Behandlungszeit durch das Pflegepersonal erreicht.

Vorzugsweise ist die erste Schicht eine Silberschicht, wodurch der geringe elektrische Widerstand in der ersten Schicht erreicht wird. Der einfließende Strom wird in der Silberschicht aufgrund ihrer guten elektrischen Leitfähigkeit homogen über die gesamte Fläche des Energieübertragungsmedium verteilt und gelangt anschließend über die zweite Schicht in die Wundauflage.

Des weiteren ergibt sich durch die Verwendung von Silber die Möglichkeit, an jeder Stelle des Energieübertragungsmediums die Stromzuführung anzuordnen, da auch bei einer seitlichen Anordnung oder beispielsweise an einer Ecke des Energieübertragungsmediums eine optimale Stromverteilung gewährleistet ist. Strommaxima treten nicht auf. Ein zusätzlicher Vorteil der Verwendung von Silber ist, dass dieses antimikrobiell wirkt.

Weiterhin ist vorgesehen, dass die Wundauflage hydrophil ist und adhäsive Eigenschaften hat, wobei die Wundauflage Hydrogel, ein Hydrokolloid, Alginat oder ein Polyurethan-Schaum sein kann. Im Gegensatz zu den bekannten inaktiven Wundauflagen, insbesondere Mullkompressen, Tupfer oder Saugvlieskompressen, werden die zuvor genannten Wundauflagen, insbesondere Hydrogele, zur Behandlung von geschädigter Haut und Wunden bevorzugt. Vorteilhaft bei ihrem Einsatz ist unter anderem, dass sie eine gute biologische Verträglichkeit aufweisen, insbesondere dann, wenn sie über einen längeren Zeitraum appliziert werden. Aufgrund der hydrophilen Eigenschaft kann die Wundauflage Flüssigkeit, wie Wundexsudat (eiweißhaltige Flüssigkeit, die bei Entzündungen aus den Gefäßen austritt), in größeren Mengen unter Volumenzunahme aufnehmen, ohne seinen Zusammenhalt zu verlieren. Insbesondere kann durch atmosphärischen Sauerstoffausschluss aus der Wunde der Heilungsprozess weiter beschleunigt werden, weil die Wunde gezwungen ist, Sauerstoff über das Blut in das Wundgebiet zu bringen. Dieses erfolgt durch vermehrte Gefäßneubildung, wodurch die Wundheilung gefördert wird. Hydrogel sowie Hydrokolloide, Alginate oder Polyurethan-Schäume stellen hierbei geeignete Mittel dar, um derartige nahezu sauer-stofffreie Bedingungen in der Wunde zu schaffen.

Bei einer weiteren bevorzugten Ausführungsform des Wundverbandes kann die Wundauflage wundheilungsfördernde Substanzen aufweisen. Hierbei sind die Substanzen vorzugsweise Wachstumsfaktoren. Während die Wundauflage auf dem zu therapierenden Wundbereich aufliegt und Wundexsudat aufnimmt, gibt sie gleichzeitig wundheilungsfördernde Stoffe an die Wunde ab, wodurch der Heilungsprozess der Wunde beschleunigt wird. Besonders bei schlecht heilenden, chronischen Wunden ist eine Wundauflage mit wundheilungsfördernden Substanzen von Vorteil. Weiterhin kommen als wundheilungsfördernde Substanzen Antibiotika, Antiseptika, Vitamine, Analgetika oder andere Substanzen infrage.

Das Energieübertragungsmedium kann als Elektrode einer Elektrostimulation dienen. Als hierfür geeignetes Material kommt Metall, ein elektrisch leitender Kunststoff, insbesondere Gummi, und Silikon in Frage.

Des weiteren kann der Wundverband an der dem Energieübertragungsmedium abgewandten Seite eine zusätzliche Schicht aufweisen, die vorzugsweise eine abziehbare Folie ist. Diese schützt die Wundauflage vor Verunreinigungen und kann vor dem Aufbringen auf die Wundfläche einfach von dem Wundverband entfernt werden. Hierbei ist es von Vorteil, wenn die Folie vorzugsweise eine Polyethylen-, Polypropylen-, oder Polyurethan-Folie ist, die wasser- und wirkstoffundurchlässig ist.

Nachstehend wird die Erfindung anhand der beigefügten Zeichnungen näher erläutert. Es zeigen
Fig. 1 eine Draufsicht eines Ausführungsbeispiels eines Wundverbandes gemäß der Erfindung,
Fig. 2 in einer rein schematischen Schnittdarstellung gemäß Linie II-II den Wundverband gemäß Fig. 1.
Fig. 3 in einer Draufsicht ein zweites Ausführungsbeispiel eines Wundverbandes gemäß der Erfindung und
Fig. 4 in einer rein schematischen Schnittdarstellung gemäß Linie III-IV den Wundverband gemäß Fig. 3.

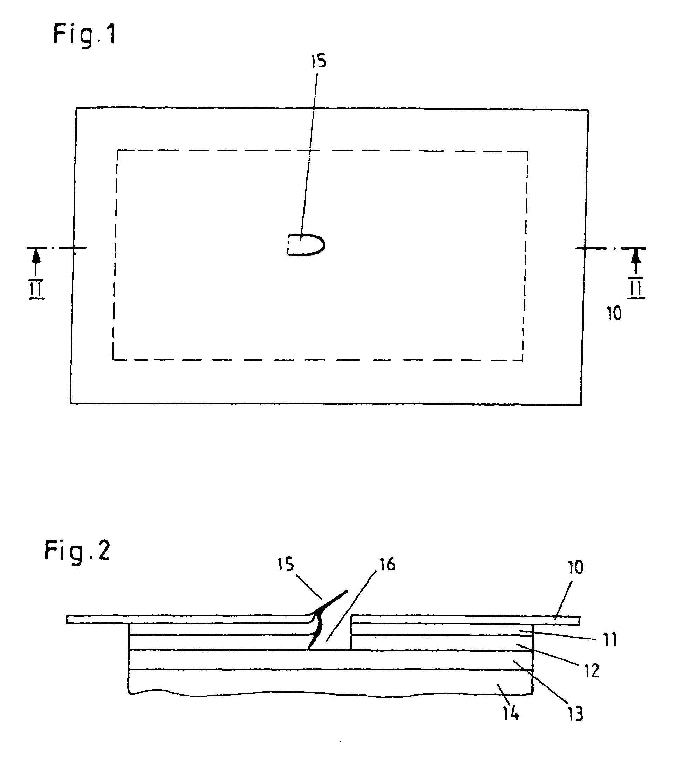

Die Figuren 1 und 2 zeigen eine bevorzugte Ausführungsform eines Wundverbandes 100. Der Wundverband 100 weist ein Energieübertragungsmedium 11,12 in Form einer zweischichtigen Elektrode auf, die mit einer ersten Energie zuführenden Schicht 11 und einer zweiten Energie verteilenden Schicht 12 ausgebildet ist. Diese Schichten 11,12 liegen mit ihren Oberflächen aufeinander und können eine Folie bilden. Um eine gleichmäßige Stromverteilung an der Oberfläche des Energieübertragungsmediums 11,12 zu gewährleisten, ist die erste Schicht 11 eine Silberschicht. Hierdurch wirkt bezweckt, dass diese Schicht 11 niederohmig ist beziehungsweise einen geringeren elektrischen Widerstand aufweist. An der der zweiten Schicht 12 abgewandten Seite ist an der ersten Schicht 11 eine Schutz/Fixierungsschicht 10 angeordnet, die das Energieübertragungsmedium 11,12 abdeckt, so dass dieses auf der umgebenden Haut fixiert ist. Hierbei besteht die nicht elektrisch leitende Schutz/Fixierungsschicht 10 aus kunststofffolienartigem pflasterfolienartigem oder schaumstofffolienartigem Material.

Damit eine elektrische Verbindung zu der durch die SchutzlFixierungsschicht 10 verdeckten Elektrode 11,12 hergestellt werden kann, ist bei der dargestellten Ausführungsform an der Schutz/Fixierungsschicht 10 eine Öffnung 16 angeordnet, welches durch die Figur 2 besonders verdeutlicht ist. Durch die Öffnung 16 verläuft ein elektrischer Kontakt 15, der aus der ersten u n d der zweiten Schicht 11/12 besteht. Wie dargestellt kann die Öffnung 16 beispielsweise als hochbiegbare Lasche ausgebildet sein. Das Ende des elektrischen Kontaktes 15 ist mit einem Energieerzeuger verbindbar, der jedoch in den Figuren nicht gezeigt ist.

Unterhalb der zweiten Schicht 12 - an der der ersten Schicht 11 abgewandten Seite - ist eine Wundauflage 13 angeordnet, welches in dem dargestellten Ausführungsbeispiel ein Gel 13 ist. Dieses Gel 13 ist hydrophil und elektrisch leitend und kann wundheilungsfördernden Substanzen enthalten. Das Gel 13 befindet sich im direkten Kontakt zur Wunde/Haut 14. Bevor der Wundverband100 jedoch auf die Wunde 14 aufgelegt wird, wird eine Folie (nicht dargestellt) abgezogen, die an der Oberfläche des Gels 13 angeordnet ist und somit das Gel 13 vor Verunreinigungen schützt. Wichtig ist, dass ein leichtes Abziehen der Folie möglich ist, ohne Bereiche aus der Wundauflage 13 mit zu entfernen. Damit der Wundverband 100, insbesondere das Gel 13, nicht Flüssigkeit oder die wundheilungsfördernden Substanzen während der Lagerung verliert, ist die Folie wasser- und wirkstoffundurchlässig ausgebildet.

Nachdem die Folie entfernt ist, wird der Wundverband 100 auf die zu behandelnde Wunde 14 aufgelegt. Das Gel 13 weist hierbei an seiner Oberfläche adhäsive Eigenschaften auf, so dass es auf der Haut/Wunde 14 - auch über längere Zeiträume - haften bleibt.

Während der Stimulationsbehandlung wird der Wundverband 100 mit Strom, insbesondere mit Impulsen, beaufschlagt. Hierbei fließt der Strom vom Energieerzeuger über den elektrischen Kontakt 15 in die erste Schicht 11 der Elektrode 11, 12. Aufgrund des geringen Widerstandes der ersten Schicht 11 verteilt sich der Strom homogen und fließt anschließend in die zweite Schicht 12 mit dem größeren Widerstand. Von dort wird die elektronische Stimulation durch das darunterliegende Gel 13, welches eine homogene elektrische Leitfähigkeit aufweist, zu der Wunde 14 weitergeleitet, so dass eine gleichmäßige Stimulation über die Wundfläche 14 erreicht wird.

Während der Stimulationsbehandlung kann das Gel 13 wundheilungsfördernde Wirkstoffe an die Wunde 14 abgeben, wodurch sich der Heilungsprozess beschleunigen lässt. Gleichzeitig ist das Gel 13 aufgrund seiner Zusammensetzung in der Lage, Wundexsudat in größeren Mengen aufzunehmen. Hierbei wird die Aufnahmekapazität an Flüssigkeit (wie Wasser oder Wundexsudat) durch Absorber sichergestellt. Als Absorber sind beispielsweise Polymere geeignet. Zudem ist das Gel 13 strukturstabil, insbesondere während der Stimulationsbehandlung der Wunde 14 und während des Verbleibens auf der Wunde 14.

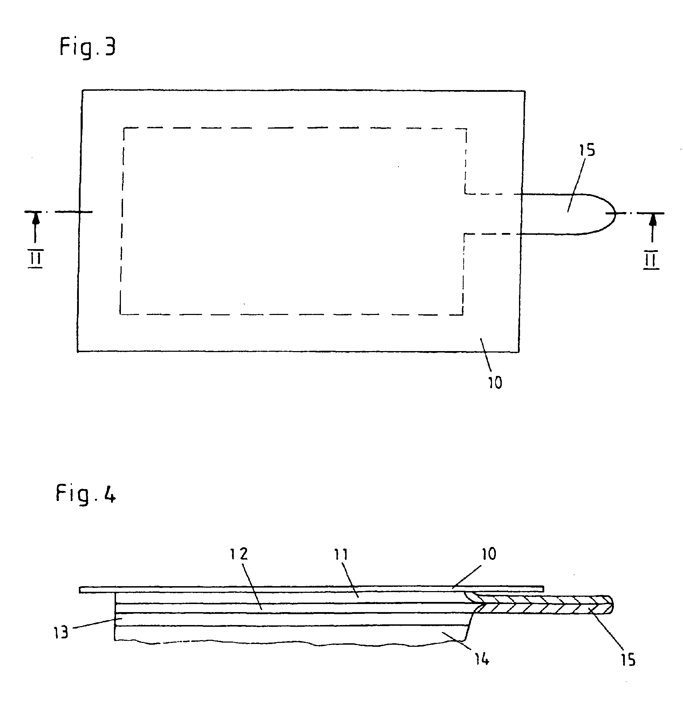

Die Figuren 3 und 4 zeigen eine weitere Ausführungsform des Wundverbandes 100. Gleiche Teile haben die gleichen Bezugszeichen wie die entsprechenden Teile bei Fig. 1 und 2 und es gilt die gleiche Beschreibung. Der Unterschied besteht in der seitlichen Anordnung des elektrischen Kontaktes 15, der als Anschlussmittel dient und der hier mit den Schichten 11, 12 verbunden seitlich herausgeführt ist.

### BEZUGSZEICHENLISTE

- 100: Wundverband
- 10: Schutz/Fixierungsschicht
- 11: erste Schicht des Energieübertragungsmediums - Energiezuführung -
- 12: zweite Schicht des Energieübertragungsmediums - Energieverteilung -
- 13: Wundauflage/Gel
- 14: Haut, Wunde
- 15: Anschlussmittel, elektrischer Kontakt
- 16: Öffnung

## Patentansprüche

1. Wundverband (100) mit mindestens einem Energieübertragungsmedium (11, 12), einer elektrisch leitenden Wundauflage (13) und einer Schutz/Fixierungsschicht (10), **dadurch gekennzeichnet, dass** das Energieübertragungsmedium (11, 12) eine Folie ist, deren erste Schicht (11) die Energiezuführung mit einer homogenen Stromverteilung über die ganze Fläche des Energieübertragungsmediums gewährleistet und deren zweite Schicht (12) zur Energieverteilung vorgesehen ist,
die erste Schicht (11 einen geringeren und die zweite Schicht (12) einen höheren elektrischen Widerstand aufweist,
die erste Schicht (11) an der der zweiten Schicht (12) abgewandten Seite die Schutz/Fixierungsschicht (10) aufweist,
die zweite Schicht (12) and der der ersten Schicht (11 abgewandten Seite die Wundauflage (13) aufweist,
und dass die Wundauflage (13) wärmeleitend und/oder bei Energiebeaufschlagung wärmeerzeugend ist.

2. Wundverband nach Anspruch 1, **dadurch gekennzeichnet, dass** die erste Schicht (11) eine Silberschicht ist.

3. Wundverband nach einem der vorhergehenden Ansprüche, **dadurch gekennzeichnet, dass** die Wundauflage (13) hydrophil ist.

4. Wundverband nach einem der vorhergehenden Ansprüche, **dadurch gekennzeichnet, dass** die Wundauflage (13) adhäsive Eigenschaften aufweist.

5. Wundverband nach einem der vorhergehenden Ansprüche, **dadurch gekennzeichnet, dass** die Wundauflage (13) z.B. ein Hydrogel, Hydrokolloid, Alginat oder ein Polyurethan-Schaum ist.

6. Wundverband nach einem der vorhergehenden Ansprüche, **dadurch gekennzeichnet, dass** die Wundauflage (13) wundheilungsfördernde Substanzen aufweist.

7. Wundverband nach Anspruch 6, **dadurch gekennzeichnet, dass** die wundheilungsfördernde Substanz ein Wachstumsfaktor ist.

8. Wundverband nach Anspruch 7, **dadurch gekennzeichnet, dass** die wundheilungs(-fördernde) Substanz Antibiotika, Antiseptika, Vitamine, Analgetika oder andere Wirkstoffe enthält.

9. Wundverband nach einem der vorhergehenden Ansprüche, **dadurch gekennzeichnet, dass** das Energieübertragungsmedium (11, 12) eine Elektrode zur Elektrostimulation ist.

10. Wundverband nach einem der vorhergehenden Ansprüche, **dadurch gekennzeichnet, dass** das Energieübertragungsmedium (11, 12) aus Metall oder elektrisch leitendem Kunststoff, insbesondere Gummi besteht.

11. Wundverband nach einem der vorhergehenden Ansprüche, **dadurch gekennzeichnet, dass** das Energieübertragungsmedium (11, 12) ein elektrisches Anschlussmittel (15) aufweist, das mit einem Energieerzeuger verbindbar ist.

12. Wundverband nach einem der vorhergehenden Ansprüche, **dadurch gekennzeichnet, dass** das Anschlussmittel (15) an der ersten Schicht (11) angeordnet ist.

13. Wundverband nach einem der vorhergehenden Ansprüche, **dadurch gekennzeichnet, dass** das Anschlussmittel (15) an der ersten und zweiten Schicht (11, 12) angeordnet ist.

14. Wundverband nach einem der vorhergehenden Ansprüche, **dadurch gekennzeichnet, dass** die Schutz/Fixierungsschicht (10) mit einer Öffnung (16) ausgebildet ist.

15. Wundverband nach einem der vorhergehenden Ansprüche, **dadurch gekennzeichnet, dass** die Schutz/Fixierungsschicht (10) aus selbst haftendem Material besteht.

16. Wundverband nach einem der vorhergehenden Ansprüche, **dadurch gekennzeichnet, dass** die Wundauflage (13) an der dem Energieübertragungsmedium (11, 12) abgewandten Seite eine zusätzliche Schicht aufweist.

17. Wundverband nach Anspruch 16, **dadurch gekennzeichnet, dass** die zusätzliche Schicht eine abziehbare Folie ist.

18. Wundverband nach Anspruch 17, **dadurch gekennzeichnet, dass** die Folie aus Polyethylen, Polypropylen oder Polyurethan besteht.

19. Wundverband nach Anspruch 17 oder 18, **dadurch gekennzeichnet, dass** die Folie (14) wasser- und wirkstoffundurchlässig ist.

20. Wundverband nach einem der vorhergehenden Ansprüche, **dadurch gekennzeichnet, dass** der Wundverband (100) flexibel und elastisch ist.

## Claims

1. Wound dressing (100) comprising at least one energy transfer medium (11, 12), an electrically conducting wound pad (13) and a protecting/fixing layer (10), **characterised in that** the energy transfer medium (11, 12) is a foil, the first layer (11) of which provides for the energy supply with a homogenous current distribution across the total area of the energy transfer medium, and the second layer (12) of which is provided for energy distribution,
the first layer (11) has a lower electric resistance and the second layer (12) a higher electric resistance,
the first layer (11) comprises the protective/fixing layer (10) at the side facing away from the second layer (12),
the second layer (12) comprises the wound pad (13) at the side facing away from the first layer (11),
and that the wound pad (13) is heat conducting and/or is generating heat when energy it applied thereto.

2. Wound dressing according to claim 1, **characterised in that** the first layer (11) is a silver layer.

3. Wound dressing according to one of the preceding claims, **characterised in that** the wound pad (13) is hydrophilic.

4. Wound dressing according to one of the preceding claims, **characterised in that** the wound pad (13) comprises adhesive properties.

5. Wound dressing according to one of the preceding claims, **characterised in that** the wound pad (13) is, for example, a hydrogel, hydrocolloid, alginate or a polyurethane foam.

6. Wound dressing according to one of the preceding claims, **characterised in that** the wound pad (13) comprises substances stimulating wound healing.

7. Wound dressing according to claim 6, **characterised in that** the substance stimulating wound healing is a growth stimulating factor.

8. Wound dressing according to claim 7, **characterised in that** the wound healing (stimulating) substance contains antibiotics, antiseptics, vitamins, analgesics or other active agents.

9. Wound dressing according to one of the preceding claims, **characterised in that** the energy transfer medium (11, 12) is an electrode for electrostimulation.

10. Wound dressing according to one of the preceding claims, **characterised in that** the energy transfer medium (11, 12) consists of metal or electrically conducting plastics, in particular rubber.

11. Wound dressing according to one of the preceding claims, **characterised in that** the energy transfer medium (11, 12) comprises an electric connecting means (15), which can be connected to an energy generator.

12. Wound dressing according to one of the preceding claims, **characterised in that** the connecting means (15) is arranged at the first layer (11).

13. Wound dressing according to one of the preceding claims, **characterised in that** the connecting means (15) is arranged at the first and second layer (11, 12).

14. Wound dressing according to one of the preceding claims, **characterised in that** the protecting/fixing layer (10) is formed with an opening (16).

15. Wound dressing according to one of the preceding claims, **characterised in that** the protecting/fixing layer (10) consists of self-adhesive material.

16. Wound dressing according to one of the preceding claims, **characterised in that** the wound pad (13) comprises an additional layer at the side facing away from the energy transfer medium (11, 12).

17. Wound dressing according to claim 16, **characterised in that** the additional layer is a strippable foil.

18. Wound dressing according to claim 17, **characterised in that** the foil consists of polyethylene, polypropylene or polyurethane.

19. Wound dressing according to claim 17 or 18, **characterised in that** the foil (14) is impermeable for water and active agents.

20. Wound dressing according to one of the preceding claims, **characterised in that** the wound dressing (100) is flexible and resilient.

## Revendications

1. Pansement avec au moins un médium de transfert d'énergie (11, 12), une couche de contact avec la plaie (13) électro-conductrice et une couche de protection/fixation (10) **caractérisé en ce que**
- le milieu de transfert d'énergie (11,12) est constitué d'une feuille dont la première couche (11) garantit l'alimentation en énergie avec une distribution de courant homogène sur toute la surface du milieu de transfert d'énergie et dont la seconde couche (12) assure la répartition de l'énergie,
- la première couche (11) comporte une résistance électrique plus faible et la seconde couche (12) une résistance électrique plus forte,
- la première couche (11) est pourvue sur sa face opposée à la seconde couche (12) d'une couche de protection/fixation (10),
- la seconde couche (12) est pourvue sur sa face opposée à la première couche (11) de la couche de contact avec la plaie (13),
- la couche de contact avec la plaie (13) est thermo-conductrice et/ou, en cas d'alimentation en énergie, thermogène.

2. Pansement selon la revendication 1, **caractérisé en ce que** la première couche (11) est une couche en argent.

3. Pansement selon l'une des revendications précédentes, **caractérisé en ce que** la couche de contact avec la plaie (13) est hydrophile.

4. Pansement selon l'une des revendications précédentes, **caractérisé en ce que** la couche de contact avec la plaie (13) présente des propriétés adhésives.

5. Pansement selon l'une des revendications précédentes, **caractérisé en ce que** la couche de contact avec la plaie (13) est constituée par exemple d'un hydrogel, d'un hydrocolloïde, d'un alginate ou d'une mousse de polyuréthane.

6. Pansement selon l'une des revendications précédentes, **caractérisé en ce que** la couche de contact avec la plaie (13) contient des substances favorisant la guérison de la plaie.

7. Pansement selon la revendication 6, **caractérisé en ce que** la substance favorisant la guérison de la plaie est un facteur de croissance.

8. Pansement selon la revendication 7, **caractérisé en ce que** la substance favorisant la guérison de la plaie contient des antibiotiques, des antiseptiques, des vitamines, des analgésiques ou d'autres substances actives.

9. Pansement selon l'une des revendications précédentes, **caractérisé en ce que** le milieu de transfert d'énergie (11,12) est constitué d'une électrode en vue d'une électro-stimulation.

10. Pansement selon l'une des revendications précédentes, **caractérisé en ce que** le milieu de transfert d'énergie (11, 12) est en métal ou en matière synthétique électro-conductrice, en particulier du caoutchouc.

11. Pansement selon l'une des revendications précédentes, **caractérisé en ce que** le milieu de transfert d'énergie (11, 12) comporte un élément de raccordement électrique (15) pouvant être relié à un dispositif de production d'énergie.

12. Pansement selon l'une des revendications précédentes, **caractérisé en ce que** l'élément de raccordement électrique (15) est disposé sur la première couche (11).

13. Pansement selon l'une des revendications précédentes, **caractérisé en ce que** l'élément de raccordement électrique (15) est disposé sur la première couche (11) et sur la seconde couche (12).

14. Pansement selon l'une des revendications précédentes, **caractérisé en ce que** la couche de protection/fixation (10) est pourvue d'un orifice (16).

15. Pansement selon l'une des revendications précédentes, **caractérisé en ce que** la couche de protection/fixation (10) est en un matériau autocollant.

16. Pansement selon l'une des revendications précédentes, **caractérisé en ce que** la couche de contact avec la plaie (13) comporte une couche supplémentaire disposée sur sa face opposée au médium de transfert d'énergie (11, 12).

17. Pansement selon la revendication 16, **caractérisé en ce que** la couche supplémentaire est une feuille retirable.

18. Pansement selon la revendication 17 **caractérisé en ce que** la feuille retirable est en polyéthylène, en polypropylène ou en polyuréthane.

19. Pansement selon les revendications 17 ou 18, **caractérisé en ce que** la feuille retirable (14) est imperméable à l'eau et aux substances actives.

20. Pansement selon l'une des revendications précédentes, **caractérisé en ce que** le pansement (100) est souple et élastique.
